# EUROPÄISCHE PATENTSCHRIFT

(11) **EP 1 423 533 B1**
(45) Veröffentlichungstag und Bekanntmachung des Hinweises auf die Patenterteilung: **20.04.2011**
(21) Anmeldenummer: 02752971.8
(22) Anmeldetag: 20.06.2002
(51) Int. Cl.: C12Q 1/68

(54) **VERFAHREN ZUM NACHWEIS VON CYTOSIN-METHYLIERUNG MIT HOHER SENSITIVITÄT**
METHOD FOR HIGH SENSITIVITY DETECTION OF CYTOSINE-METHYLATION
PROCEDE TRES SENSIBLE POUR DETECTER LA METHYLATION DE LA CYTOSINE

(30) Priorität: 22.06.2001 DE 10130800
(43) Veröffentlichungstag der Anmeldung: 02.06.2004
(73) Patentinhaber: Epigenomics AG, 10178 Berlin (DE)
(72) Erfinder: BERLIN, Kurt, 14532 Stahnsdorf (DE)
(86) Internationale Anmeldenummer: PCT/DE2002/002264
(87) Internationale Veröffentlichungsnummer: WO 2003/000926

(56) Entgegenhaltungen:
- WO-A-00/70090
- WO-A-99/28498
- WO-A-99/55905
- US-A- 6 143 504
- XIONG Z AND LAIRD P W: "COBRA: a sensitive and quantitative DNA methylation assay" NUCLEIC ACIDS RESEARCH, OXFORD UNIVERSITY PRESS, SURREY, GB, Bd. 25, Nr. 12, 1997, Seiten 2532-2534, XP002106407 ISSN: 0305-1048 in der Anmeldung erwähnt
- SADRI RAMIN ET AL: "Rapid analysis of DNA methylation using new restriction enzyme sites created by bisulfite modification" NUCLEIC ACIDS RESEARCH, OXFORD UNIVERSITY PRESS, SURREY, GB, Bd. 24, Nr. 24, 1996, Seiten 5058-5059, XP002192857 ISSN: 0305-1048
- REIN ET AL: "Identifying 5-methylcytosine and related modifications in DNA genomes" NUCLEIC ACIDS RESEARCH, OXFORD UNIVERSITY PRESS, SURREY, GB, Bd. 26, Nr. 10, 1998, Seiten 2255-2264, XP002143106 ISSN: 0305-1048 in der Anmeldung erwähnt
- OLEK A ET AL: "A modified an improved method for bisulphite based cytosine methylation analysis" NUCLEIC ACIDS RESEARCH, OXFORD UNIVERSITY PRESS, SURREY, GB, Bd. 24, Nr. 24, 1996, Seiten 5064-5066, XP002106408 ISSN: 0305-1048 in der Anmeldung erwähnt
- GONZALGO M L AND JONES P A: "Rapid quantitation of methylation differences at specific sites using methylation-sensitive single nucleotide primer extension (Ms-SNuPE)" NUCLEIC ACIDS RESEARCH, OXFORD UNIVERSITY PRESS, SURREY, GB, Bd. 25, Nr. 12, 15. Juni 1997 (1997-06-15), Seiten 2529-2531, XP002089928 ISSN: 0305-1048 in der Anmeldung erwähnt

## Beschreibung

Die vorliegende Offenbarung betrifft ein Verfahren zum Nachweis von Cytosin-Methylierung in DNA-Proben. Das Verfahren dient insbesondere zum Nachweis der Anwesenheit oder Abwesenheit von Cytosin-Methylierung in zu untersuchender DNA in Proben eines Individuums, in denen nicht zu untersuchende Hintergrund DNA des gleichen Individuums vorliegt, die sich nur hinsichtlich des Methylierungsstatus von der zu untersuchenden DNA unterscheidet.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene bzw. des Genoms korrelierbar. Insofern äußern sich pathogene Zustände in einem veränderten Methylierungsmuster einzelner Gene oder des Genoms.

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Eine relativ neue und die mittlerweile am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällung- und Reinigungsschritte durch schnelle Dialyse ersetzt (Olek A, Oswald J, Walter J. A modified and improved method for bisulphate based cytosine methylation analysis. Nucleic Acids Res. 1996 DEC 15;24(24):5064-6). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannten Möglichkeiten, 5-Methylcytosine nachzuweisen, kann aus dem folgenden Übersichtsartikel entnommen werden:Rein T, DePamphilis ML, Zorbas H. Identifying 5-methylcytosine and related modifications in DNA genomes. Nucleic Acids Res. 1998 May 15;26(10):2255-64.

Die Bisulfit-Technik wird bisher bis auf wenige Ausnahmen (z. B. Zeschnigk M, Lich C, Buiting K, Dörfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) nur in der Forschung angewendet. Immer aber werden kurze, spezifische Stücke eines bekannten Gens nach einer Bisulfit-Behandlung amplifiziert und entweder komplett sequenziert (Olek A, Walter J. The pre-implantation ontogeny of the H19 methylation imprint. Nat Genet. 1997 Nov.;17(3):275-6) oder einzelne Cytosin-Positionen durch eine "Primer-Extension-Reaktion" (Gonzalgo ML, Jones PA. Rapid quantitation of methylation differences at specific sites using methylation-sensitive single nucleotide primer extension (Ms-SNuPE). Nucleic Acids Res. 1997 Jun. 15;25(12):2529-31, WO-Patent 9500669) oder einen Enzymschnitt (Xiong Z, Laird PW. COBRA: a sensitive and quantitative DNA methylation assay. Nucleic Acids Res. 1997 Jun. 15;25(12):2532-4) nachgewiesen. Zudem ist auch der Nachweis durch Hybridisierung beschrieben worden (Olek et al., WO 99 28498).

Harnstoff verbessert die Effizienz der Bisulfit-Behandlung vor der Sequenzierung von 5-Methylcytosin in genomischer DNA (Paulin R, Grigg GW, Davey MW, Piper AA. Urea improves efficiency of bisulphate-mediated sequencing of 5'-methylcytosine in genomic DNA. Nucleic Acids Res. 1998 Nov. 1;26(21):5009-10).

Weitere Publikationen, die sich mit der Anwendung der Bisulfit-Technik zum Methylierungsnachweis bei einzelnen Genen befassen, sind:
Grigg G, Clark S. sequencing 5-methylcytosine residues in genomic DNA. Bioassays. 1994 Jun.;16(6):431-6, 431; Zeschnigk M, Schmitz B, Dittrich B, Buiting K, Horsthemke B, Dörfler W. Imprinted segments in the human genome: different DNA methylation patterns in the Prader-Willi/Angelman syndrome region as determined by the genomic sequencing method. Hum Mol Genet. 1997 Mar;6(3) :387-95; Feil R, Charlton J, Bird AP, Walter J, Reik W. Methylation analysis on individual chromosomes: improved protocol fort bisulphate genomic sequencing. Nucleic Acids Res. 1994 Feb. 25;22(4) :695-6; Martin V, Ribieras S, Song-Wang X, Rio MC, Dante R. Genomic sequencing indicates a correlation between DNA hypomethylation in the 5' region of the pS2 gene andin its expression in human breast cancer cell lines. Gene. 1995 May 19;157(1-2):261-4; WO97/46705 WO95/15373 und WO97/45560.

Ein weiteres bekanntes Verfahren ist die sogenannte methylierungssensitive PCR (Herman JG, Graff JR, Myohanen S, Nelkin BD, Baylin SB. (1996), Methylation-specific PCR: a novel PCR assay for methylation status of CpG islands. Proc Natl Acad Sci U S A. Sep 3;93(18):9821-6). Für dieses Verfahren werden Primer verwendet, die entweder nur an eine Sequenz hybridisieren, die durch die Bisulfit-Behandlung einer an der betreffenden Position unmethylierten DNA entsteht, oder aber umgekehrt Primer, welche nur an eine Nukleinsäure bindet, die durch die Bisulfit-Behandlung einer an der betreffenden Position unmethylierten DNA entsteht. Mit diesen Primer können demnach Amplifikate erzeugt werden, deren Detektion wiederum Hinweise auf das Vorliegen einer methylierten oder unmethylierten Position in der Probe liefern, an welche die Primer binden.

Ein weiteres MSP-Verfahren zur Analyse des fragilen X-Syndroms ist in dem US-Patent US 6,143,505 beschrieben.

Ein neueres Verfahren ist auch der Nachweis von Cytosin-Methylierung mittels einer Taqman PCR, das als Methyl-Light bekannt geworden ist (WO00/70090). Mit diesem Verfahren ist es möglich, den Methylierungsstatus einzelner oder weniger Positionen direkt im Verlauf der PCR nachzuweisen, so dass sich eine nachfolgende Analyse der Produkte erübrigt.

Eine Übersicht über den Stand der Technik in der Oligomer Array Herstellung lässt sich aus einer im Januar 1999 erschienenen Sonderausgabe von Nature Genetics (Nature Genetics Supplement, Volume 21, January 1999), der dort zitierten Literatur und dem US-Patent 5994065 über Methoden zur Herstellung von festen Trägern für Zielmoleküle wie Oligonucleotide bei vermindertem nichtspezifischen Hintergrundsignal entnehmen.

Für die Abtastung eines immobilisierten DNA-Arrays sind vielfach fluoresziert markierte Sonden verwendet worden. Besonders geeignet für Fluoreszenzmarkierungen ist das einfache Anbringen von Cy3 und Cy5 Farbstoffen am 5'-OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Matrix-assistierte Laser Desorptions/Ionisations-Massenspektrometrie (MALDI-TOF) ist eine sehr leistungsfähige Entwicklung für die Analyse von Biomolekülen (Karas M, Hillenkamp F. Laser desorption ionization of proteins with molecular masses exceeding 10,000 daltons. Anal Chem. 1988 Oct. 15;60(20):2299-301). Ein Analyt wird in eine lichtabsorbierende Matrix eingebettet. Durch ei-nen kurzen Laserpuls wird die Matrix verdampft und das Analytmolekül so unfragmentiert in die Gasphase befördert. Durch Stöße mit Matrixmolekülen wird die Ionisation des Analyten erreicht. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere.

MALDI-TOF Spektroskopie eignet sich ausgezeichnet zur Analyse von Peptiden und Proteinen. Die Analyse von Nukleinsäuren ist etwas schwieriger (Gut, I. G. und Beck, S. (1995), DNA and Matrix Assisted Laser Desorption Ionization Mass Spectrometry. Molecular Biology: Current Innovations and Future Trends 1: 147-157.) Für Nukleinsäuren ist die Empfindlichkeit etwa 100 mal schlechter als für Peptide und nimmt mit zunehmender Fragmentgröße überproportional ab. Für Nukleinsäuren, die ein vielfach negativ geladenes Rückgrat haben, ist der Ionisationsprozeß durch die Matrix wesentlich ineffizienter. In der MALDI-TOF Spektroskopie spielt die Wahl der Matrix eine eminent wichtige Rolle. Für die Desorption von Peptiden sind einige sehr leistungsfähige Matrices gefunden worden, die eine sehr feine Kristallisation ergeben. Für DNA gibt es zwar mittlerweile einige ansprechende Matrices, jedoch wurde dadurch der Empfindlichkeitsunterschied nicht verringert. Der Empfindlichkeitsunterschied kann verringert werden, indem die DNA chemisch so modifiziert wird, dass sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln (Gut, I. G. und Beck, S. (1995), A procedure for selective DNA alkylation and detection by mass spectrometry. Nucleic Acids Res. 23: 1367-1373). Die Kopplung eines "charge tags" an diese modifizierte DNA resultiert in der Steigerung der Empfindlichkeit um den gleichen Betrag, wie er für Peptide gefunden wird. Ein weiterer Vorteil von "charge tagging" ist die erhöhte Stabilität der Analyse gegen Verunreinigungen, die den Nachweis unmodifizierter Substrate stark erschweren.

Genomische DNA wird durch Standardmethoden aus DNA von Zell-, Gewebe- oder sonstigen Versuchsproben gewonnen. Diese Standardmethodik findet sich in Referenzen wie Fritsch und Maniatis, Molecular Cloning: A Laboratory Manual, 1989.

Nach der Erfindung der PCR sind in den folgenden Jahren zahlreiche Varianten bekannt geworden; die diese Technik zur Amplifikation der DNA verfeinern. Insbesondere ist hier die Multiplexierung der PCR (Multiplex-PCR) zu erwähnen, wobei man mehr als 2 spezifische Primer einsetzt und dabei in einem Reaktionsgefäß eine Vielzahl von verschiedenen, spezifische n Amplifikation erzeugen kann. Besonders interessant ist auch die sogenannte Nested PCR, welche unter anderem zum Nachweis besonders geringer DNA Mengen verwendet wird. Diese Art der PCR besteht aus zwei aufeinanderfolgenden Amplifikationen, wobei die Primer der zweiten Amplifikation innerhalb des ersten Amplifikates liegen und nicht mit den Primern der ersten Amplifikation identisch sind. Dadurch wird eine besondere Spezifität erreicht, da die Primer der zweiten Amplifikation nur dann funktionieren, wenn in der ersten Amplifikation das beabsichtigte Fragment erzeugt wurde. Dagegen ist die die Vermehrung etwaiger Nebenprodukte der ersten Amplifikation in der zweiten so gut wie ausgeschlossen.

Es sind demnach bislang vielerlei Verfahren zur Methylierungsanalyse Stand der Technik. Die vorliegende Offenbarung soll jedoch das Problem lösen, dass die gängigen Verfahren nicht in der Lage sind, eine in einer Körperflüssigkeit oder Serum befindliche zu untersuchende DNA gezielt zu amplifizieren, wenn zugleich andere, sequenzhomologe DNA-Abschnitte anderen Ursprungs zugegen sind.

Dies wäre aber besonders vorteilhaft, da beispielsweise im Serum sich freie DNA aus unterschiedlichsten Quellen befinden kann. Da DNA aus unterschiedlichen Quellen in einem Individuum sich normalerweise nicht in der Sequenz, jedoch durchaus im Methylierungsgmuster unterscheidet (wenn man mal von etwa vorhandener viraler oder bakterieller DNA absieht), besteht der Bedarf nach einem Verfahren, welches die DNA bevorzugt anreichert und damit der genauen Methylierungsanalyse zugänglich macht, welche aus einer ganz bestimmten Quelle stammt. Dies ist besonders bedeutsam für den Nachweis abweichender Methylierungsmuster in Tumoren, die auf diese Art aus beispielsweise Serum nachgeweisen werden können.

Die zu untersuchende DNA sowie die ansonsten vorhandenen, im folgenden Hintergrund-DNA genannten Nukleinsäuren, werden in aller Regel gleichermaßen amplifiziert, da die verwendeten Primer auch nicht in der Lage sind, zwischen zu untersuchender DNA und Hintergrund-DNA zu unterscheiden. Eine Möglichkeit zur Unterscheidung dieser DNAs ergibt sich jedoch durch das unterschiedliche Methylierungsmuster. Ein gängiges Verfahren ist die methylierungssensitive PCR, kurz MSP (Herman JG, Graff JR, Myohanen S, Nelkin BD, Baylin SB. (1996), Methylation-specific PCR: a novel PCR assay for methylation status of CpG islands. Proc Natl Acad Sci U S A. Sep 3;93(18):9821-6). Dieses Verfahren besteht aus mehreren Teilschritten. Zunächst wird eine dem Stand der Technik entsprechende Bisulfit-Behandlung durchgeführt, welche wiederum dazu führt, dass alle Cytosinbasen in Uracil umgewandelt werden, während die methylierten Cytosinbasen (5-Methylcytosin) unverändert bleiben. Im nächsten Schritt verwendet man nun Primer, welche vollständig komplementär zu einer methylierten, mit Bisulfit umgewandelten DNA sind, nicht jedoch zu einer entsprechenden DNA welche ursprünglich nicht methyliert vorlag. Das führt bei der Durchführung einer PCR mit einem solchen Primer dazu, dass ausschließlich die ursprünglich methylierte DNA amplifiziert wird. Entsprechend ist es möglich, einen Primer zu verwenden, der im Gegenzug nur die unmethylierte DNA amplifiziert. Auf diese Art und Weise können, wenn zu analysierende DNA sowie Hintergrund DNA zugegen sind, ausschließlich die zu untersuchenden DNA Fragmente selektiv erzeugt werden, sofern sich diese hinsichtlich ihres Methylierungsstatus in einer CpG Position von der Hintergrund DNA unterscheiden.

Stand der Technik ist es nun, aus dem Nachweis eines solchen zu untersuchenden DNA-Moleküls auf den Methylierungszustand oder das Vorliegen einer zu untersuchenden DNA rückzuschließen, was wiederum eine Diagnose beispielsweise einer Tumorerkrankung in Patienten prinzipiell erlaubt, da es bekannt ist, das beispielsweise die Serum DNA-Konzentration sich in Tumorpatienten zum Teil drastisch erhöht. Nur die von den Tumoren stammende DNA soll dann neben der Hintergrund-DNA nachgewiesen werden. Prinzipiell vergleichbar ist die Analyse von DNA in anderen Körperflüssigkeiten.

Stand der Technik ist wiederum ein von Epigenomics entwickeltes Verfahren, welches zu untersuchende DNA und Hintergrund-DNA nach Bisulfit-Behandlung gleichermaßen amplifiziert und dann die im Fragment enthaltenen ehemaligen CpG Positionen durch Hybridisierungstechniken untersucht, alternativ mittels MiniSequenzierung oder anderen gängigen Verfahren. Dies hat den Vorteil, dass man ein quantitatives Bild bezüglich der untersuchten Methylierungspositionen erhält, d. h. es erfolgt die Bestimmung des Methylierungsgrades einer Vielzahl von Positionen, was z. B. bei soliden Tumoren eine sehr genau Klassifizierung ermöglicht. Der Nachteil dieser Methode ist jedoch, dass sie in den Fällen, in denen die Hintergrund-DNA stark überwiegt, keine genau Aussage liefern kann, da diese ja genau wie die zu untersuchende DNA amplifiziert wird und beide im Gemisch analysiert werden. Dieses Problem existiert nicht bei der Analyse von soliden Tumoren, wo man das zu untersuchende Material gezielt auswählen kann, es kann jedoch die Analyse von beispielsweise Serum-DNA erschweren.

Ziel der vorliegenden Offenbarung ist es nun, die Nachteile des Standes der Technik zu überwinden und die Vorteile beider Verfahren für die Detektion von Methylierungsmustern in Körperflüssigkeiten und Serum zu kombinieren. Wie oben erwähnt ist dies besonders bedeutsam für den Nachweis abweichender Methylierungsmuster in Tumoren, die auf diese Art aus beispielsweise Serum nachgewiesen werden können.

Die Aufgabe wird dadurch gelöst, dass ein Verfahren zum Nachweis von Cytosin-Methylierung in DNA-Proben geschaffen wird bei dem dass man die folgenden Schritte ausführt :
man behandelt eine genomische DNA-Probe, welche zu untersuchende DNA und Hintergrund-DNA umfasst, chemisch, bevorzugt mit einem Bisulfit (=Disulfit, Hydrogensulfit), derart, dass alle nicht methylierten Cytosinbasen in Uracil umgewandelt werden, während die 5-Methylcytosinbasen unverändert bleiben;
man amplifiziert die chemisch behandelte DNA-Probe unter Verwendung von bevorzugt mindestens 2 Primeroligonukleotiden mittels einer Polymerasereaktion,
die DNA wird enzymatisch selektiv an solchen Positionen geschnitten, die in der DNA Probe einen Methylierungsstatus aufweisen, welcher fuer die weiter zu untersuchende DNA nicht charakteristisch ist, nicht aber für Hintergrund-DNA;
die nicht enzymatisch geschnittene DNA wird in einer weiteren Polymeraseraktion amplifiziert, dadurch wird die zu untersuchende DNA gegenüber etwa vorhandener Hintergrund DNA angereichert;
das Amplifikat wird hinsichtlich seiner Sequenzeigenschaften untersucht und daraus auf den Methylierungsstatus in der zu untersuchenden DNA in der genomischen DNA-Probe geschlossen.

Bevorzugt ist es, dass man die Proben DNA aus Serum oder anderen Körperflüssigkeiten eines Individuums gewinnt.

Es ist weiterhin bevorzugt, dass man die Proben DNA aus Zellinien, Blut, Sputum, Stuhl, Urin, Serum, Gehirn-Rückenmarks-Flüssigkeit, in Paraffin einbettetem Gewebe, beispielsweise Gewebe von Darm, Niere, Hirn, Herz, Prostata, Lunge, Augen, Brust oder Leber, histologischen Objektträgern und allen möglichen Kombinationen hiervon gewinnt.

Es ist ganz besonders bevorzugt, dass man die chemische Behandlung mit einem Bisulfit (=Disulfit, Hydrogensulfit) durchführt. Bevorzugt ist es auch, dass die chemische Behandlung nach Einbetten der DNA in Agarose erfolgt. Es ist auch und weiterhin bevorzugt, dass bei der chemischen Behandlung ein die DNA-Duplex denaturierendes Reagenz und/oder ein Radikalfänger zugegen ist.

Besonders bevorzugt ist es auch, die Amplifikationen mehrerer unterschiedlicher Fragmente mit mehr als 2 verschiedenen Primern in einem Reaktionsgefäß durchzuführen und damit die Amplifikationsschritte als Multiplex-PCR auszuführen. Es ist generell besonders bevorzugt, die Amplifikationen als Polymerasekettenreaktion auszuführen.

Die in den Amplifikationen verwendeten Primer amplifizieren besonders bevorzugt keine Fragmente aus nicht mit Bisulfit behandelter genomischer DNA (oder nur in vernachlässigbarem Ausmaß), so dass sie für die mit Bisulfit umgewandelte DNA spezifisch sind. Dies schützt vor fehlerhaften Ergebnissen im Falle einer unvollständigen Umwandlungsreaktion mit beispielsweise Natriumbisulfit.

Der enzymatische Schnitt der Hintergrund-DNA wird besonders bevorzugt mit einer Restriktionsendonuklease oder mehreren unterschiedlichen Restriktionsenzymen ausgeführt. Werden mehrere Restriktionsendonuklease n verwendet, so hängt es von den jeweiligen Puffern ab, ob diese nacheinander oder gleichzeitig zum Einsatz kommen. Dem Fachmann ist die Anwendung von Restriktionsenzymen gemäß den von den Herstellern mitgelieferten Protokollen bekannt. Die hier im folgenden als bevorzugt aufgelisteten Restriktionsenzyme, welche kommerziell erhältlich sind, erheben nicht den Anspruch auf Vollständigkeit: Mae II, Psp 1406 I, Ast II, Ssp 5230 I, Bbr P I, Bsa Al, Sna B I, Cfo I, Hin P1 I, Eco 47 III, NAR I, Ehe I, Kas I, Bbe I, Hae II, Acy I, Ban I, Hgi CI, Aos I, Avi II, Hpa II, Msp I, Pin AI, Age I, Eco 56 I, Nae I, Cfr10I, SgrAI, Fse I, XmaCI, Sma I, Srf I, Ava I, Bse Al, Mro I, Taq I, Cla I, Sal I, Hind II, Acc I, Xho I, Sfu I, BstBI, Hinf I, Sau 96 I, Dra II, PssI, Ita I, Dsa V, Scr F I, Mae III, Bst E II, Dde I, Cel II, Esp I oder Aoc I.

Die Restriktionsenzyme schneiden, da sie nach der Bisulfit Umwandlung zwischen TG und CG bzw. auf dem Gegenstrang zwischen CG und CA Dinukleotiden unterscheiden müssen, allesamt Sequenzen die eines dieser Motive beinhalten.

Demnach schneiden die Restriktionsendonukleasen besonders bevorzugt entweder an Positionen, die vor der Bisulfit Umwandlung und Amplifikation einer im wesentlichen methylierten CpG Position in der zu untersuchenden DNA entsprachen, während die Hintergrund-DNA an dieser Position im wesentlichen unmethyliert vorlag und/oder die Restriktionsendonukleasen schneiden an Positionen, die die vor der Bisulfit Umwandlung und Amplifikation einer im wesentlichen unmethylierten CpG Position in der zu untersuchenden DNA entsprachen, während die Hintergrund-DNA an dieser Position im wesentlichen methyliert vorlag.

In einer besonders bevorzugten Variante des Verfahrens werden in dem Restriktionsschritt mindestens 90% aller in der vorherigen Amplifikation erzeugten Fragmente geschnitten. Dies ist bei entsprechender Vollständigkeit dieses enzymatischen Schrittes vor allem dann der Fall, wenn die zu untersuchende DNA weniger als 10% der Gesamt-DNA ausmacht. Dies ist jedoch besonders bevorzugt, da dann die Vorteile der hier vorgestellten Methode gegenüber herkömmlichen Techniken besonders zu Tragen kommen, die hohe Spezifität aufgrund der zwei unterschiedlichen Amplifikationen zusammen mit der hohen Selektivität für die zu untersuchende DNA.

Besonders bevorzugt werden daher in dem zweiten Amplifikationsschritt zusätzlich oder ausschliesslich Primer verwendet, die an die Amplifikate des ersten Schrittes hybridisieren, nicht aber mit den Primern des ersten Amplifikationsschrittes hybridisieren oder zu ihnen in wesentlichen Abschnitten sequenzhomolog sind. Damit wird in diesem Verfahren eine nested PCR durchgeführt, wobei zwischen den Amplifikationsschritten die Fragmente enzymatisch geschnitten werden, die man der Hintergrund-DNA zuordnet. Diese Fragmente können dann in der folgenden Amplifikation nicht mehr als Template für eine PCR dienen und demzufolge wird ausschliesslich die zu untersuchende DNA amplifiziert. Besonders bevorzugt, damit dies der Fall ist, liegen daher die Restriktionsschnittstellen innerhalb der auch in dem zweiten Amplifikation zu vermehrenden Sequenzabschnitt. Dennoch ist auch eine Variante des Verfahrens bevorzugt, in der der gleiche Satz von Primern in beiden Amplifikationsschritten verwendet wird. Dies ist insbesondere dann vorteilhaft, wenn hochgradig multiplexierte PCR durchgeführt wird, da die Etablierung dieser Reaktionen aufwendig ist und man sich auf diese Art die zweifache Etablierung eines dazugehörigen Primersatzes mit den entsprechenden Reaktionsbedingungen spart. Dies geht dann zu Lasten der Spezifität dieser Amplifikationen.

Es ist besonders bevorzugt, dass der erste Amplifikationsschritt als Multiplex-PCR ausgeführt wird. Besonders bevorzugt ist auch die Ausführung beider Amplifikationsschritte als Multiplex-PCR.

Besonders bevorzugt ist auch eine Variante, in der die Primer des zweiten Amplifikationsschrittes mit den Schnittstellen der Restriktionsendonuklease(n) überlappen. In diesem Fall wird die Hybridisation der Primer in diesem Schritt an die geschittenen Amplifikate von vorneherein verhindert was erneut die spezifische Amplifikation der aus der zu untersuchenden DNA hervorgegangenen Fragmente fördert.

Bevorzugt ist es ferner, dass die Hintergrund-DNA in 100 facher Konzentration im Vergleich zur zu untersuchenden DNA vorliegt. Weiterhin ist bevorzugt, dass die Hintergrund-DNA in 1000 facher Konzentration im Vergleich zur zu untersuchenden DNA vorliegt.

Bevorzugt ist es ferner, dass die Analyse, oder gegebenenfalls die weitere Analyse mittels Hybridisierung an Oligomer-Arrays erfolgt, wobei Oligomere Nukleinsäuren oder in ihren Hybridisierungseigenschaften ähnliche Moleküle wie PNAs sein können.

Vorteilhaft ist es auch, dass die Oligomere über einen 12-22 Basen langen Abschnitt an die zu analysierende DNA hybridisieren und sie ein CG, TG oder CA Dinukleotid umfassen.

Es ist bevorzugt, dass der Methylierungsstatus von mehr als 10 Methylierungspositionen der zu analysierenden DNA in einem Experiment nachgewiesen wird.

Weiterhin ist bevorzugt, dass der Methylierungsstatus von mehr als 60 Methylierungspositionen der zu analysierenden DNA in einem Experiment nachgewiesen wird.

Auch ist es bevorzugt, dass die Analyse, oder gegebenenfalls die weitere Analyse durch Längenmessung der amplifizierten zu untersuchenden DNA erfolgt, wobei Methoden zur Längenmessung Gelelektrophorese, Kapillargelelektrophorese, Chromatographie (z.B. HPLC), Massenspektrometrie und andere geeignete Methoden umfassen. Vorteilhaft ist es dabei auch, dass Methoden zur Sequenzierung die Sanger-Methode, Maxam-Gilbert-Methode und andere Methoden wie Sequencing by Hybridisation (SBH) umfassen.

Bevorzugt ist auch ein Verfahren, wobei man die Sequenzierung für jede oder eine kleine Gruppe von CpG Positionen mit jeweils einem separaten Primeroligonukleotid ausführt und die Verlängerung der Primer nur eine oder einige wenige Basen ausmacht, und man aus der Art der Primerverlängerung auf den Methylierungsstatus der betreffenden Positionen in der zu untersuchenden DNA schließt.

Weiterhin ist bevorzugt, dass man aus dem Methylierungsgrad an den verschiedenen untersuchten CpG Positionen auf das Vorliegen einer Erkrankung oder eines anderen medizinischen Zustandes des Patienten schließt.

Vorteilhaft ist es, dass die Amplifikate selbst für die Detektion mit einer nachweisbaren Markierung versehen sind. Bevorzugt werden diese Markierungen entweder durch eine Markierung der Primer oder der Nukleotide während der Amplifikation in die erzeugten Fragmente eingebracht.

Weiterhin vorteilhaft ist es, dass die Markierungen Fluoreszenzmarkierungen sind. oder/und dass die Markierungen Radionuklide sind oder/und dass die Markierungen ablösbare Massenmarkierungen sind, die in einem Massenspektrometer nachgewiesen werden.

Bevorzugt ist ferner, dass bei der Amplifikation einer der Primer an eine Festphase gebunden ist. Bei diesen Festphasen kann es sich beispielsweise um funktionalisierte Polymere, Metalle, Glas oder Halbleiter wie Silicium handeln. Das Anbinden der Primer erfolgt bevorzugt über binfunktionale Linkermoleküle, welche an eine silanisierte Oberfläche gebunden werden oder aber beispielsweise über Thioate in den Primer an Bromacetylderivatisierte Oberflächen oder Gold.

Ist es auch, dass die Amplifikate insgesamt im Massenspektrometer nachgewiesen werden und somit durch ihre Masse eindeutig charakterisiert sind.

Besonders bevorzugt ist es auch, die Bildung spezifischer Fragmente während der Amplifikation unter Verwednung von Reporteroligonukleotiden zu beobachten, welche ihre Fluoreszenzeigenschaften durch spezifische Interaktion mit dem jeweiligen Amplflikat und anderen Oligonukleotiden, den Primern und/oder der Polymerase ändern.

Dabei ist es vorteilhaft, dass man zusätzlich zu dem Reporteroligonukleotid ein weiteres mit einem Fluoreszenzfarbstoff markiertes Oligomer verwendet, welches unmittelbar benachbart zu dem Reporteroligonukleotid hybridisiert und sich diese Hybridisierung mittels Fluoreszenz Resonanz Energietransfer nachweisen lässt. Weiterhin vorteilhaft ist es, dass ein Taqman-Assay durchgeführt wird. Bevorzugt ist es auch, dass ein Lightcycler Assay durchgeführt wird. Weiterhin ist bevorzugt, dass die Reporteroligonukleotide mindestens eine Fluoreszenzmarkierung tragen. Ferner ist auch bevorzugt, dass die Reportermoleküle die Amplifikation entweder durch eine Zunahme oder eine Abnahme der Fluoreszenz anzeigen. Dabei ist es besonders vorteilhaft, dass man die Zunahme oder Abnahme der Fluoreszenz auch direkt zur Analyse verwendet und aus dem Fluorenzenzsignal auf einen Methylierungszustand der zu analysierenden DNA schließt.

Ein weiterer Gegenstand der vorliegenden Offenbarung ist auch die Verwendung eines offenbarten Verfahrens zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

Vorteilhaft ist dabei die Verwendung eines offenbartengsgemäßen Verfahrens zur Unterscheidung von Zelltypen oder Geweben oder zur Untersuchung der Zelldifferenzierung.

Gegenstand der vorliegenden Offenbarung ist auch ein Kit, bestehend aus einem Bisulfit enthaltenen Reagenz, Primern zur Herstellung der Amplifikate, sowie optional einer Anleitung zur Durchführung eines offenbarungsgemäßen Assays.

Die vorliegende Offenbarung somit ein Verfahren zur Detektion des Methylierungszustandes genomischer DNA Proben. Im Gegensatz zu bislang bekannten Verfahren wird der Methylierungsgrad eines Satzes von CpG Positionen in einer ausgewählten Untergruppe von DNA-Fragmenten z. B. in Serum bestimmt, so dass eine Analyse auch in Gegenwart eines Überschusses an diagnostisch nicht relevanter Hintergrund-DNA möglich ist.

Die im zweiten Amplifikationsschritt erhaltenen Fragmente werden auf ihre Methylierungssignatur hin analysiert und der Methylierungsgrad bevorzugt mehrerer ehemaliger CpG Positionen in den Amplifikaten bestimmt. Bevorzugt wird aus dem Methylierungsgrad an den verschiedenen untersuchten CpG Positionen auf das Vorliegen einer Erkankung oder eines anderen medizinischen Zustandes des Patienten geschlossen.

Das Wesen der vorliegenden Offenbarung ist es nun, dass zwei Arten von CpG Positionen eine Rolle spielen und gleichermaßen zur Analyse beitragen und die nachfolgend als "Qualifier"-Positionen und "Classifier"-Positionen benannt werden sollen. Die Qualifier-Positionen dienen dazu, zwischen den beiden Amplifikationsschritten, bei der enzymatischen Spaltung, zwischen der zu analysierende DNA und der Hintergrund-DNA zu unterscheiden. Dies kann technisch auf unterschiedliche Art und Weise erfolgen. Eigenschaft dieser Positionen ist es jedoch, dass ihr Methylierungsgrad in zu untersuchender DNA möglichst verschieden ist von dem in der Hintergrund-DNA. Die Classifier-Positionen dienen im Gegensatz dazu, aus dem Amplifikat, erzeugt überwiegend aus der zu untersuchenden DNA, über den jeweiligen Methylierungsgrad die für die Diagnose bedeutende Information zu extrahieren. Es können bis zu einigen 100 solcher Classifier Positionen für eine Analyse verwendet werden, erfolgt die Analyse beipielsweise auf Oligomer-Arrays, auch wenn dies oftmals nicht erforderlich sein wird. Es ist jedoch in diesem Fall weniger das Entstehen eines bestimmten Amplifikates, das für das Untersuchungsergebnis von Bedeutung ist, sondern vielmehr die Analyse der CpG-Positionen in selbigem Amplifikat. Dies unterscheidet die hier beschriebene Methode grundlegend von anderen sonst bekannten, für die Methylierungsanalyse genutzten Methoden wie MSP. In einigen Fällen ist es jedoch sicher möglich und sinnvoll, die aus dem Entstehen eines Amplifikates abzuleitende Information in die Analyse mit einzubeziehen, in diesem Fall sind einige Positionen dann zugleich Classifier und Qualifier.

Ein besonders bevozugtes mögliches Vorgehen ist im folgenden beschrieben.

Der erste Schritt des Verfahrens, die Gewinnung von Proben, erfolgt bevorzugt durch Entnahme von Körperflüssigkeiten wie z. B. Sputum oder aber Serum, jedoch ist es offenkundig dass das Verfahren mit vielerlei Proben aus unterschiedlichen Quellen durchführbar ist.

Es erfolgt in einigen Fällen vor der Bisulfit-Behandlung eine Aufreinigung oder Aufkonzentration der DNA, um eine Störung der Bisulfit-Reaktion und/oder der nachfolgenden PCR durch ein zu hohes Maß an Verunreinigungen zu vermeiden. Es ist jedoch bekannt, dass beipielsweise eine PCR aus Gewebe nach Behandlung beispielsweise mit Proteinase K ohne weitere Aufreinigung erfolgen kann, und dies gilt sinngemäß auch für die Bisulfit-Behandlung und nachfolgende PCR.

Die chemische Behandlung wird bevorzugt durch Behandlung mit einem Bisulfit (=Hydrogensulft, Disulfit), wiederum bevorzugt Natriumbisulfit (weniger geeignet ist Ammoniumbisulfit) durchgeführt. Entweder erfolgt die Reaktion nach einer publizierten Variante, bevorzugt ist hier die Einbettung der DNA in Agarose, um die DNA während der Behandlung in einzelsträngigen Zustand zu halten, oder aber nach einer neuen Variante durch Behandlung in Gegenwart eines Radikalfängers und eines denaturierenden Reagenzes, bevorzugt ein Oligoethylenglykoldialkylether oder beipielsweise Dioxan. Vor der PCR Reaktion werden die Reagenzien entweder durch Waschen im Falle der Agarosemethode oder einem DNA-Aufreinigungsverfahren (Stand der Technik, Fällung oder Bindung an eine Festphase, Membran) entfernt oder aber einfach durch Verdünnung in einen Konzentrationsbereich gebracht, der die PCR nicht mehr signifikant beeinflusst.

Wesentlich für den nächsten Schritt ist es nun, dass die Qualifier Positionen ausgewählt werden und ein geeignetes Restriktionsenzym gewählt wird, das selektive Spaltung der nicht zu analysierenden Hintergrund-DNA erlaubt. Die Auswahl der Positionen erfolgt nach der Prämisse, dass sie sich zwischen Hintergrund-DNA und zu untersuchender DNA hinsichtlich ihrer Methylierung so sehr wie möglich unterscheiden sollten, zudem muss für den entsprechenden Sequenzkontext ein geeignetes Enzym verfügbar sein. Das Restriktionsenzym muss zudem so ausgewählt werden, dass sich keine Schnitte etwa in anderen gewünschten Amplifikaten ergeben.
Zunächst werden also die Methylierungsprofile der jeweils in Frage kommenden Abschnitte eines Gens sowohl für die zu untersuchenden Tumore als auch für die Hintergrund-DNA aus gesunden Individuen bestimmt. Diejenigen Positionen, die die größten Unterschiede zwischen Tumor DNA und Hintergrund-DNA (beispielsweise im Serum) aufweisen, werden als Qualifier Positionen ausgewählt. Solche Positionen sind für eine Vielzahl von Genen bereits bekannt, beipielsweise für GSTpi, für HIC-1 und MGMT (von Wronski MA, Harris LC, Tano K, Mitra S, Bigner DD, Brent TP. (1992) Cytosine methylation and suppression of O6-methylguanine-DNA methyltransferase expression in human rhabdomyosarcoma cell lines and xenografts. Oncol Res.;4(4-5):167-74; Esteller M, Toyota M, Sanchez-Cespedes M, Capella G, Peinado MA, Watkins DN, Issa JP, Sidransky D, Baylin SB, Herman JG. (2000), Inactivation of the DNA repair gene 06-methylguanine-DNA methyltransferase by promoter hypermethylation is associated with G to A mutations in K-ras in colorectal tumorigenesis. Cancer Res. May 1; 60 (9) :2368-71).

Die chemisch behandelte DNA wird im Prinzip, wie es Stand der Technik ist, mittels mindestens zweier Primer amplifiziert, eine Multiplexierung ist möglich. Innerhalb des von den beiden Primern eingegrenzten DNA-Abschnittes befinden sich eine oder mehrere Qualifier-Positionen, aber bevorzugt auch eine oder mehrere Classifier-Positionen. Die Reihenfolge dieser Positionen ist für das Aufsetzen des hier beschriebenen Assays unerheblich.

Wie oben aufgeführt, wird nach der ersten Amplifikation ein Verdau mit einem oder mehreren Restriktionsenzymen, je nach Anzahl der Qualifier-Positionen, durchgeführt. Nach dem Verdau werden die noch vorhandenen Primer, Enzyme und eventuell Nukeotide entweder durch einen Aufreinigungsschritt entfernt (beipielsweise kann eine Ethanol-Präzipitierung durchgeführt werden, oder es wird ein handelsüblicher Aufreinigungskit für PCR Produkte verwendet) oder aber die Lösung wird mit PCR-Puffer für die zweite Amplifikation so erheblich verdünnt, dass die oben genannten Bestandteile in dieser Reaktion nicht ins Gewicht fallen.

Wie oben angesprochen, können mehrere Qualifier-Positionen und auch demnach mehrere, jeweils für einen in der Hintergrund-DNA vorliegenden Methylierungsstatus und damit nach Bisulfit-Umwandlung für eine bestimmte Sequenz spezifische Restriktionsenzyme in einem solchen Verfahren verwendet werden.

Die Primer der zweiten Amplifikation liegen bevorzugt innerhalb des in der ersten Amplifikation erzeugten Fragmentes und zwar derart, dass sie mit den zuvor verwendeten Primern weder signifikant überlappen noch mit ihnen hybridisieren. Es wird also eine nested PCR durchgführt. Damit das Verfahren funktioniert, ist darauf zu achten, dass die Qualifier Positionen innerhalb des zweiten Amplifikates liegen. Eine Überlappung der Primer der zweiten Amplifikation mit der Qualifier-Position ist möglich, wenn diese sich so nahe am 3'-Ende des Primers befindet, dass im Falle geschnittener DNA keine Amplifikation mehr stattfinden kann.

Nach der selektiven, zweiten Amplifikation der zu untersuchenden DNA können nun bevorzugt, nach an sich bekannten Verfahren, der Methylierungsstatus mehrerer Classifier-Positionen bestimmt werden.

Es ist offensichtlich, dass auch in diesem Fall das Entstehen eines PCR Fragmentes selbst im Einzelfall von hinreichender Aussagekraft sein kann, sofern man, wie auch bei der MSP, die Situation vorliegen hat, dass die Qualifier-Position praktisch zu 100% beispielsweise in der Hintergrund-DNA unmethyliert vorliegt, jedoch in der zu untersuchenden DNA aufmethyliert vorliegt. Verwendet man nun in der PCR ein Oligonukleotid, das bevorzugt an die Sequenz bindet, welche in der Bisulfit-Behandlung aus nicht methylierter Hintergrund-DNA entsteht, so entsteht in der PCR nur dann ein Produkt, wenn wenigstens eine geringe Menge an zu untersuchender DNA überhaupt vorhanden ist. Dies kann im Einzelfall bereits hinreichend für eine Diagnose sein, und es würde sich hierbei um ein Verfahren handeln, dass ein ähnliches Anwendungspotential aufweist wie MSP. Obgleich eine solche Durchführung nicht direkt bevorzugt ist, ist ein solches Verfahren bislang nicht bekannt und wird demzufolge ebenfalls als zugehörig zum Gegenstand dieser Offenbarung betrachtet.

Bevorzugt ist es, dass in einer PCR-Reaktion mehrere Fragmente gleichzeitig erzeugt werden, d.h. dass eine Multiplex-PCR durchgeführt wird. Bei bisulfit-behandelter DNA hat man dabei den Vorteil, dass aufgrund des unterschiedlichen G und C-Gehaltes der beiden DNA-Stränge ein Forward-Primer niemals auch als Reverse-Primer fungieren kann, was die Multiplexierung erleichtert.

Im einfachsten Fall werden nun die entstandenen Fragmente nachgewiesen, ohne Einzelinformation über den Methylierungsgrad der in ihnen vorhanden ehemaligen CpG Positionen zu erhalten. Dazu kommen alle möglichen bekannten molekularbiologischen Verfahren in Frage, wie Gelelektrophorese, Sequenzierung, Flüssigchromatographie oder Hybridisierungen, ohne dass dabei die Classifier Positionen separat analysiert werden. Dies wäre auch zur Qualitätskontrolle der vorangehenden Verfahrensschritte denkbar. Wie oben ausgeführt, ist jedoch die nachfolgende Analyse des Methylierungsgrades der Classifier-Positionen besonders bevorzugt.

Es gibt zahlreiche Möglichkeiten die bevorzugte Amplifikation der zu untersuchenden DNA im zweiten Amplifikationsschritt vorteilhaft mit Detektionstechniken für die Classifier-Oligonukleotide zu kombinieren.

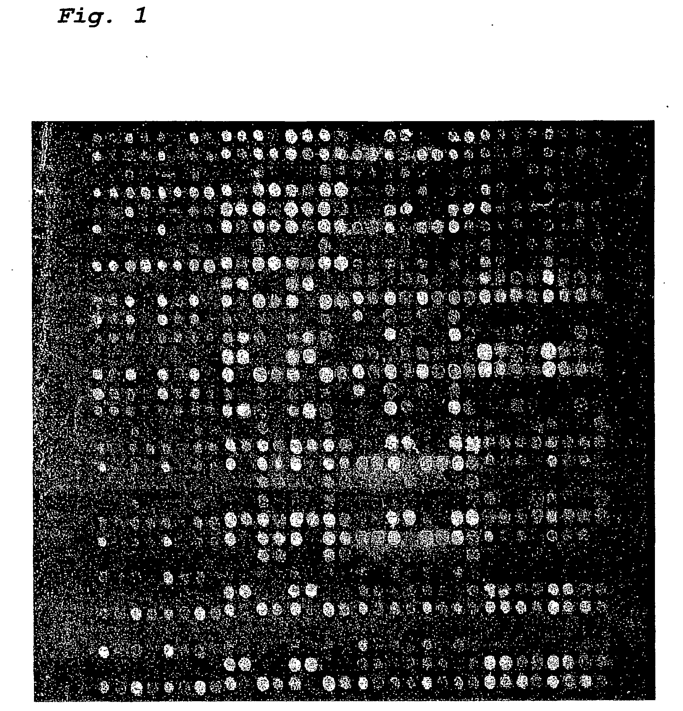

Detektionstechniken, die sich besonders eignen, sind die Hybridisierung an Oligomerarrays und beispielsweise Primer-Extension (MiniSequenzierung) Reaktionen. Die Hybridisierung an Oligomerarrays kann ohne weitere Veränderung von Protokollen gegenüber dem nächstliegenden Stand der Technik verwendet werden (Olek A, Olek S, Walter J; WO-Patent 9928498). Jedoch ist es bevorzugt, die Amplifikate an einen Array von Oligomeren zu hybridisieren, der aus Paaren von an einer Festphase immobilisierten Oligonukleotiden besteht, von denen eines jeweils stark bevorzugt an einen ein ursprünglich unmethyliertes CpG (Classifier-Position) enthaltenden DNA-Abschnitt hybridisiert und das andere wiederum stark bevorzugt an den entsprechenden Abschnitt, in dem ursprünglich ein methyliertes CpG enthalten war, jeweils vor der Bisulfit-Behandlung und Amplifikation. Besonders bevorzugt ist in diesem Fall das Amplifikat oder die Amplifikate fluoreszent oder radioaktiv oder mit ablösbaren Massentags markiert, so dass sich nach der Hybridisierung die an die beiden Oligonukleotide eines Paares gebundenen Fragmente anhand dieser Markierung nachweisen und quantifizieren lassen. Man erhält ein Intensitätsverhältnis, aus dem man beispielsweise nach Eichung des Experimentes mit vollständig methylierter und unmethylierter DNA den Methylierungsgrad an der jeweiligen Classifier-Position bestimmen kann. Auf einem solchen Oligomer-Array (Fig. 1) lassen sich eine Vielzahl von Fragmenten und Classifier-Positionen gleichzeitig nachweisen.
Es ist sinnvoll und bevorzugt, dass der Array auch Qualifier-Positionen detektierende Oligomere zur Kontrolle des Experimentes enthält, da so das Verhältnis der in die Analyse eingehenden zu untersuchenden DNA zur Hintergrund-DNA bestimmt werden kann.

Primerextensionsreaktionen können ebenfalls an auf einer Festphase immobilisierten Oligonukleotide ausgeführt werden. Obgleich nicht zwingend erforderlich, ist die Immobilisierung dieser Primer bevorzugt, da in der Regel eine Vielzahl von Classifier-Positionen aus mehreren Amplifikaten untersucht werden soll und dies auf einer Festphase, also an einem Oligomerarrays, bedeutend leichter und in einem Experiment durchführbar ist. Es ist besonders bevorzugt, dass die Primer sich unmittelbar neben einer Classifier-Position befinden und dass die Verlängerung nur um ein Nukleotid erfolgt. Es ist besonders bevorzugt, dass lediglich Didesoxythymidin und Didesoxycytidin als Nukleotide zugesetzt werden und dass diese jeweils mit einem unterschiedlichen Fluoreszenzfarbstoff markiert sind, wobei allerdings auch andere, unterscheidbare Markierungen wie Massentags denkbar und bevorzugt sind. Nach einer Bisulfit-Behandlung und Amplifikation liegen ehemalige methylierte CG als CG und nicht methylierte CG nunmehr als TG vor. Die Primerextensionsreaktion führt daher entweder zum Einbau eines Didesoxycytidins oder Didesoxythymidins. Aus dem Verhältnis der für diese beiden Terminatoren jeweils detektierten Fluoreszenzmarkierungen lässt sich auf den Methylierungsgrad der jeweiligen Position schließen. Es ist auch möglich und bevorzugt, in diesem Fall die Primerextension mit Desoxycytidin und Desoxythymidin durchzuführen, wenn man kein Guaninderivat zugibt und demzufolge bei einer TG oder CG Sequenz bereits nach einer Base die Primerextension ohnehin endet. Zudem ist es ebenfalls bevorzugt, die Analyse auf dem Gegenstrang durch Unterscheidung von CA und CG analog durchzuführen, dann entsprechend mit Didesoxy-ATP und Didesoxy-GTP oder deren Derivaten.

Eine besonders bevorzugte Variante des Verfahrens ist jedoch die gleichzeitige Detektion von Qualifier-Positionen und Classifier-Positionen in einem Experiment, was sich durch Verwendung von Taqman oder Lightcycler-Technologievarianten (Real time PCR) erzielen lässt. In diesem Sonderfall des vorliegenden Verfahrens wird die zweite Amplifikation als Real time PCR mit entsprechenden Reporteroligonukleotiden durchgeführt, die an verschieden Classifier Positionen binden können. Dabei werden bevorzugt Paare solcher Reporteroligonukleotide verwendet, wobei das eine Oligonukleotide bevorzugt an die Sequenz bindet, die einer methylierten Position vor der Bisulfit-Behandlung entspricht, und das andere an die Sequenz enstehend aus einer entsprechend unmethylierten Position. Dabei werden zusätzlich zu den Oligonukleotiden, die für eine Amplifikation der zu untersuchenden DNA sorgen, weitere fluoreszenzmarkierte Oligonukleotide zugesetzt, und die Änderung der Fluoreszenz während der PCR-Reaktion gemessen. Dabei erhält man überwiegend, weil ja die zu untersuchende DNA amplifiziert wird, auch aus dieser Fluoreszenzänderung unmittelbar Information über den Methylierungsstatus verschiedener Classifier CpG Positionen.

Da verschiedene Oligonukleotide bevorzugt jeweils mit unterschiedlichen Fluoreszenzfarbstoffen versehen werden, ist auch eine Unterscheidung der Fluoreszenzänderung währed der PCR getrennt für verschiedene Positionen möglich.

Diese vom Methylierungsstatus abhängige Fluoreszenzänderung kann durch zahlreiche Methoden erzielt werden, von denen hier beispielhaft zwei aufgeführt werden sollen.

Zum einen können Oligonukleotid Sonden verwendet werden, die spezifisch-entweder an eine Sequenz binden, die durch chemische Behandlung aus einer an der entsprechenden Position unmethylierten DNA hervorgegangen ist, oder aber entsprechend an einer Sequenz, die durch chemische Behandlung aus einer an der entsprechenden Position methylierten DNA hervorgegangen ist. Diese Sonden sind besonders bevorzugt mit zwei Fluoreszenzfarbstoffen versehen, einem Quencherfarbstoff und einem als Marker dienenden Fluoreszenzfarbstoff. Beide sind mit der gleichen Oligonukleotidsonde verknüpft. Findet nun eine PCR-Reaktion mit der zu untersuchenden DNA als Templat statt, so wird die PCR Reaktion diesmal durch die fluoreszent markierte Oligomersonde blockiert. Da diese jedoch nicht gegen die Nukleaseaktivität der Polymerase resistent ist, findet ein Abbau der an die Templat-DNA gebundenen Sonde während der PCR-Reaktion statt, der mit der Bindungseffizienz der Sonde an das Templat korreliert, da die nicht gebundene Sonde von der Polymerase nicht abgebaut wird. Der Abbau der Sonde wird nun dadurch, dass dabei der Quencherfarbstoff und der als Marker dienende Fluoreszenzfarbstoff voneinander getrennt werden, durch eine Zunahme der Fluoreszenz des Markerfarbstoffs unmittelbar sichtbar. Im Prinzip handelt es sich hierbei um eine Variante des sogenannten Taqman Assays.

Was man demnach misst, ist das Entstehen eines PCR Produktes aus der zu untersuchenden DNA, jedoch nur dann wenn die untersuchte Classifier-Position auch in dem Methylierungszustand vorliegt, den die Sonde durch Hybridisieren an die chemisch behandelte DNA detektieren kann. Eine Gegenprobe mit einer Sonde, die entsprechend an die Classifier Position im anderen Methylierungszustand binden würde, ist daher zweckmäßig und bevorzugt. Es kann prinzipiell auch nur mit einer Sonde gearbeitet werden, welche noch nicht einmal unbedingt an eine Classifier Position binden muss, und aus dem Entstehen des PCR Produktes unmittelbar nur auf das Vorliegen eines bestimmten Methylierungsstatus in der Qualifier Position geschlossen werden.

Bevorzugt werden verschiedene Fluoreszenzfarbstoffe mit unterschiedlichen Emissionswellenlängen an mehreren Sonden zusammen mit dem Quencher eingesetzt, um eine Unterscheidbarkeit der Sonden und damit eine Multiplexierung zu erreichen.

Bevorzugt ist es auch, dass mit einer Sonde mehrere Positionen gleichzeitig auf ihren Methylierungsgrad untersucht werden können.

Ist eine genauere Quantifizierung des Methylierungsgrades der Classifier-Positionen wünschenswert, so können bevorzugt auch zwei mit einander konkurrierende Sonden mit unterschiedlichen Farbstoffen eingesetzt werden, wobei eine wiederum im Fall einer unmethylierten Position in der zu untersuchenden DNA, die andere umgekehrt im Falle einer methylierten Position bevorzugt bindet. Aus dem Verhältnis der Fluoreszenzzunahmen für die beiden Farbstoffe lässt sich dann wiederum auf den Methylierungsgrad der untersuchten Position schließen.

Ein grundsätzlich anderes Verfahren, bei dem jedoch auch während der PCR eine Fluoreszenzänderung erfolgt, ist gegenwärtig als Lightcycler™ Technologie bekannt. Dabei wird ausgenutzt, das ein Fluoreszenz Resonanz Energietransfer (FRET) zwischen zwei Farbstoffen nur erfolgen kann, wenn diese sich in unmittelbarer Nähe, das heißt 1-5 Nukleotide voneinander entfernt befinden. Nur dann kann der zweite Farbstoff von der Emission des ersten Farbstoffes angeregt werden und dann seinerseits Licht einer anderen Wellenlänge emittieren, das dann detektiert wird.

Im vorliegenden Fall der Methylierungsanalyse erfolgt eine Hybridisierung einer fluoreszenzmarkierten Sonde an die betreffende chemisch behandelte DNA an einer Classifier-Position, und die Bindung dieser Sonde hängt wiederum davon ab, ob die zu untersuchende DNA an dieser Position methyliert oder unmethyliert vorlag. Unmittelbar benachbart zu dieser Sonde bindet eine weitere Sonde mit einem anderen Fluoreszenzfarbstoff. Diese Bindung erfolgt bevorzugt wiederum Sequenz- und damit methylierungsabhängig, wenn in dem betreffenden Sequenzabschnitt eine weitere methylierbare Position vorliegt (immer jeweils nach Bisulfit-Behandlung). Während der Amplifikation wird nun die DNA vermehrt, weshalb immer mehr fluoreszenzmarkierte Sonden benachbart an die betreffende Position binden, sofern diese vor der Bisulfit-Behandlung den dafür erforderlichen Methylierungszustand aufwies, und daher ein zunehmender FRET gemessen wird.

Auch bei diesem Verfahren erfolgt bevorzugt eine Multiplexierung mit mehreren verschieden fluoreszenzmarkierten Sonden.

Beide Verfahren unterscheiden sich im Ergebnis hauptsächlich darin, dass in einem Fall eine Abnahme, im anderen Fall eine Zunahme der Fluoreszenz gemessen wird. In beiden Fällen können sowohl Qualifier- als auch Classifier-Positionen gemessen werden.

Die folgenden Beispiele erläutern die Offenbarung:

### Beispiel 1:

### Durchführung des Verfahrens am Beispiel des ELK-1 Gens

Die aus Serum isolierte DNA wird unter Verwendung von Bisulphit (Hydrogensulfit, Disulfit) derart behandelt, dass alle an der 5-Position der Base nicht methylierten Cytosine in Uracil umgewandelt werden, während die in 5-Position methylierten Cytosine unverändert bleiben. Für diese Reaktion wird die im Stand der Technik bekannte und oben beschriebene Agarose-Methode verwendet. Die erste Amplifikation eines definierten Fragmentes der Länge 530 bp aus der Promotorregion des ELK-1 Gens erfolgt nun mittels zweier Primeroligonukleotiden
ATGGTTTTGTTTAATYGTAGAGTTGTTT (SEQ-ID: 1) und
TAAACCCR CCCAATAT (SEQ-ID: 2). Um alle in der 29. ehemaligen genomischen CpG Position dieses Fragmentes methylierten Amplifikate zu entfernen, wird das Amplifikat zunächst durch Ethanol Präzipitation isoliert und anschliessend mit Mae II nach den Angaben des Herstellers (Roche Molecular Biochemicals) inkubiert. Nach erfolgtem Verdau wird die Lösung 1:10000 mit PCR-Puffer verdünnt und eine zweite Amplifikation mit den Primeroligonukleotiden TTTATTTTTATATAAGTTTTGTTT (SEQ-ID: 3)und CCCTTCCCTACAAAACTATAC (SEQ-ID: 4) durchgeführt. Diese Primeroligonukleotide sind mit dem Fluoreszenfarbstoff Cy5 markiert, daher ist auch das bei der PCR erhaltene Fragment markiert.

### Beispiel 2:

### Durchführung der Hybridisierung und Auswertung eines hybridisierten DNA-"Chip"

Das in Beispiel 1 hergestellte Amplifikat wird auf einen DNA Chip hybridisiert. Auf dem Chip sind zuvor Oligonukleotide immobilisiert worden. Die Oligonukleotidesequenzen leiten sich von dem in Beispiel 1 genannten amplifizierten Fragment des Gens ELK-1 ab, und repräsentierten die CG Dinukleotide, die unmittelbare Umgebung einschließend. Die Länge der Oligonukleotide beträgt 14-22 Nukleotide, die Position des CG Dinukleotides innerhalb der Oligonukleotide ist variabel. Nach der Hybridisierung (5 h, 38°C, 5x SSC) wird der DNA Chip auf einem Fluoreszenzscanner (Genepix 4000A) vermessen und die Hybridisierungssignale numerisch ausgewertet.

Die beigefügten Figuren dienen gleichfalls zur Erläuterung der Offenbarung.

In Figur 1 ist ein DNA Chip nach Hybridisierung mit dem Promotor Fragment dargestellt. Dargestellt ist das Falschfarben-Bild wie es nach dem Scannen erzeugt wird. Entgegen der hier dargestellten schwarz-weiß Abbildung wird von dem Scanner ein farbiges Bild erzeugt. Die Intensität der verschiedenen Farben repräsentiert den Grad der Hybridisierung, wobei der Hybridisierungsgrad von rot (in Figur 1 als helle Spots zu erkennen) nach blau (in Figur 1 als dunkle Spots zu erkennen) abnimmt.

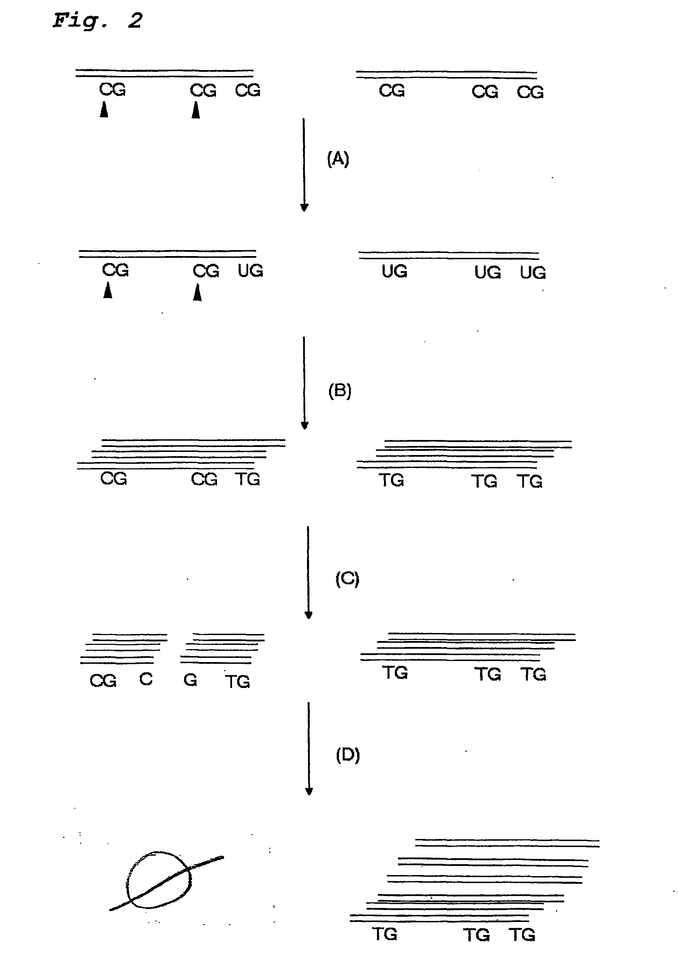

Figur 2 zeigt schematisch die grundlegende Vorgehensweise des offenbarungsgemässen Verfahrens. Auf der linken Seite ist die nicht zu analysierende Hintergrund-DNA dargestellt, die in diesem Beispiel partiell methyliert vorliegt, auf der rechten Seite entsprechend unmethylierte zu analysierende DNA. Im ersten Schritt (A) findet eine Bisulfit-Umwandlung statt, im zweiten Schritt (B) erfolgt die erste Amplifikation. Im dritten Schritt (C) wird die nicht zu analysierende Hintergrund-DNA gespalten, die verbleibende DNA wird anschliessend (D) erneut amplifiziert. Ihre Sequenzeigenschaften erlauben nachfolgend Rückschlüsse auf den Methylierungsstaus der zu untersuchenden DNA an prinzipiell allen Postionen, die sich in dem amplifizierten Bereich befinden.

## Patentansprüche

1. Verfahren zum Nachweis von Cytosin-Methylierung in DNA-Proben, **dadurch gekennzeichnet, dass** die folgenden Verfahrensschritte ausgeführt werden:
a) eine genomische DNA Probe wird chemisch, bevorzugt mit einem Bisulfit (=Disulfit, Hydrogensulfit), derart behandelt, dass Cytosin in eine vom Basenpaarungsverhalten in der DNA-Duplex her unterschiedliche Base umgewandelt wird, während 5-Methylcytosin unverändert bleibt,
b) Abschnitte der Proben-DNA werden mittels einer Polyrnerasereaktion amplifiziert,
c) die DNA wird enzymatisch selektiv an solchen Positionen geschnitten, die in der DNA Probe einen Methylierungsstatus aufwiesen, welcher für die Hintergrund-D NA charakteristisch ist, nicht aber für die weiter zu untersuchende DNA,
d) die nicht enzymatisch geschnittene DNA wird in einer weiteren Polymeraseraktion amplifiziert, **dadurch** wird die zu untersuchende DNA gegenueber etwa vorhandener Hintergrund DNA angereichert,
e) das Amplifikat wird hinsichtlich seiner Sequenz untersucht und daraus auf den Methylierungsstatus in der zu untersuchenden DNA in der genomischen DNA-Probe geschlossen.

2. Verfahren nach Anspruch 1, **dadurch gekennzeichnet, dass** man die Proben DNA aus Serum oder anderen Körperflüssigkeiten eines Individuums gewinnt.

3. Verfahren nach Anspruch 1, **dadurch gekennzeichnet, dass** man die Proben DNA aus Zellinien, Blut, Sputum, Stuhl, Urin, Serum, Gehim-Rückenmarks-Flüssigkeit, in Paraffin einbettetem Gewebe, beispielsweise Gewebe von Augen, Darm, Niere, Hirn, Herz, Prostata, Lunge, Brust oder Leber, histologischen Objektträgern und allen möglichen Kombinationen hiervon gewinnt.

4. Verfahren nach einem der voranstehenden Ansprüche, **dadurch gekennzeichnet, dass** man die chemische Behandlung mit einem Bisulfit (=disulfid, Hydrogensulfit) durchführt.

5. Verfahren nach Anspruch 4, **dadurch gekennzeichnet, dass** die chemische Behandlung nach Einbetten der DNA in Agarose erfolgt.

6. Verfahren nach Anspruch 4, **dadurch gekennzeichnet, dass** bei der chemischen Behandlung ein die DNA-Duplex denaturierendes Reagenz und/oder ein Radikalfänger zugegen ist.

7. Verfahren nach einem der voranstehenden Ansprüche, **dadurch gekennzeichnet, dass** die Amplifikation mehrerer Fragmente in einem Reaktionsgefäß in Form einer Multiplex-PCR durchgeführt wird.

8. Verfahren nach einem der voranstehenden Ansprüche, **dadurch gekennzeichnet, dass** die in der Amplifikation eingesetzten Primer die chemisch mit Bisulfit umgewandelte DNA amplifizieren, nicht aber die entsprechende nicht umgewandelte genomische Sequenz.

9. Verfahren nach einem der voranstehenden Ansprüche, **dadurch gekennzeichnet, dass** der enzymatische Schnitt mittels einer Restriktionsendonuklease erfolgt.

10. Verfahren nach Anspruch 9, **dadurch gekennzeichnet, dass** es sich bei der Restriktionsendonuklease um Mae II, Psp 1406 I, Ast II, Ssp 5230 I, Bbr PI, Bsa Al, Sna B I, Cfo I, Hin P1 I, Eco 47 III, NAR I, Ehe I, Kas I, Bbe I, Hae II, Acy I, Ban I, Hgi Cl, Aos I, Avi II, Hpa II, Msp I, Pin Al, Age I, Eco 56 I, Nae I, Cfr101, SgrAl, Fse I, XmaCl, Sma I, Srt I, Ava I, Bse Al, Mro I, Taq I, Cla I, Sal I, Hind II, Acc I, Xho I, Sfu I, BstBI, Hinf I, Sau 96 I, Dra II, PssI, Ita I, Dsa V, Scr F I, Mae III, Bst E II, Dde I, Cel II, Esp I oder Aoc I handelt.

11. Verfahren nach Anspruch 9, **dadurch gekennzeichnet, dass** mehrere Restriktionsendonukleasen zum Einsatz kommen.

12. Verfahren nach Anspruch 11, **dadurch gekennzeichnet, dass** mehrere verschiedene Restriktionsendonukleasen in einem Reaktionsgefäß zu Einsatz kommen.

13. Verfahren nach einem der voranstehenden Ansprüche, **dadurch gekennzeichnet, dass** in dem zweiten Amplifikationsschritt die gleichen Primer verwendet werden wie im ersten Amplifikationsschritt.

14. Verfahren nach einem der voranstehenden Ansprüche, **dadurch gekennzeichnet, dass** in dem zweiten Amplifikationsschritt zusätzlich oder ausschliesslich Primer verwendet werden, die an die Amplifikate des ersten Schrittes hybridisieren, nicht aber wesentlich mit den Primern des ersten Schrittes identisch sind oder mit ihnen hybridisieren (nested PCR).

15. Verfahren nach einem der voranstehenden Ansprüche, **dadurch gekennzeichnet, dass** auch der zweite Amplifikationsschritt als Multiplex PCR ausgeführt wird.

16. Verfahren nach einem der voranstehenden Ansprüche, **dadurch gekennzeichnet, dass** Primer des zweiten Amplifikationsschrittes mit den Schnittstellen der im vorangehenden Schritt eingesetzten Restriktionsendonuklease(n) überlappen.

17. Verfahren nach einem der voranstehenden Ansprüche, **dadurch gekennzeichnet, dass** die Hintergrund-DNA in 100 facher Konzentration im Vergleich zur zu untersuchenden DNA vorliegt.

18. Verfahren nach einem der voranstehenden Ansprüche, **dadurch gekennzeichnet, dass** die Hintergrund-DNA in 1000 facher Konzentration im Vergleich zur zu untersuchenden DNA vorliegt.

19. Verfahren nach einem der voranstehenden Ansprüche, **dadurch gekennzeichnet, dass** die Analyse der Sequenzeigenschaften der Amplifikate mittels Hybridisierung an Oligomer-Arrays erfolgt, wobei Oligomere Nukleinsäuren oder in ihren Hybridisierungseigenschaften ähnliche Moleküle wie PNAs sein können.

20. Verfahren nach Anspruch 19, **dadurch gekennzeichnet, dass** die Oligomere über einen 12-22 Basen langen Abschnitt an die zu analysierende DNA hybridisieren und sie ein CG, TG oder CA Dinukleotid umfassen.

21. Verfahren nach einem der Ansprüche 19 oder 20, **dadurch gekennzeichnet, dass** der Methylierungsstatus von mehr als 10 Methylierungspositionen der zu analysierenden DNA in einem Experiment nachgewiesen wird.

22. Verfahren nach einem der Ansprüche 19 oder 20, **dadurch gekennzeichnet, dass** der Methylierungsstatus von mehr als 60 Methylierungspositionen der zu analysierenden DNA in einem Experiment nachgewiesen wird.

23. Verfahren nach einem der Ansprüche 1 bis 18, **dadurch gekennzeichnet, dass** die Analyse durch Sequenzierung erfolgt, wobei Methoden zur Sequenzierung die Sanger-Methode, Maxam-Gilbert-Methode und andere Methoden wie Sequencing by Hybridisation (SBH) umfassen.

24. Verfahren nach Anspruch 23, **dadurch gekennzeichnet, dass** man die Sequenzierung für jede oder eine kleine Gruppe von CpG Positionen mit jeweils einem separaten Primeroligonukleotid ausführt und die Verlängerung der Primer nur eine oder einige wenige Basen ausmacht, und man aus der Art der Primerverlängerung auf den Methylierungsstatus der betreffenden Positionen in der zu untersuchenden DNA schließt.

25. Verfahren nach einem der voranstehenden Ansprüche, **dadurch gekennzeichnet, dass** man aus dem Methylierungsgrad an den verschiedenen untersuchten CpG Positionen auf das Vorliegen einer Erkankung oder eines anderen medizinischen Zustandes des Patienten schließt.

26. Verfahren nach einem der voranstehenden Ansprüche, **dadurch gekennzeichnet, dass** die Amplifikate selbst für die Detektion mit mindestens einer nachweisbaren Markierung versehen sind, die bevorzugt entweder durch Markierung der Primer oder aber der Nukleotide während der Amplifikation eingebracht wird.

27. Verfahren nach Anspruch 26, **dadurch gekennzeichnet, dass** die Markierungen Fluoreszenzmarkierungen sind.

28. Verfahren nach Anspruch 26, **dadurch gekennzeichnet, dass** die Markierungen Radionuklide sind.

29. Verfahren nach Anspruch 26, **dadurch gekennzeichnet, dass** die Markierungen ablösbare Massenmarkierungen sind, die in einem Massenspektrometer nachgewiesen werden.

30. Verfahren nach einem der voranstehenden Ansprüche, **dadurch gekennzeichnet, dass** bei mindestens einer der Amplifikationen einer der jeweiligen Primer an eine Festphase gebunden ist.

31. Verfahren nach einem der Ansprüche 1 bis 18, **dadurch gekennzeichnet, dass** die Amplifikate insgesamt im Massenspektrometer nachgewiesen werden und somit durch ihre Masse eindeutig charakterisiert sind.

32. Verfahren nach einem der Ansprüche 1 bis 18, **dadurch gekennzeichnet, dass** die Bildung spezifischer Fragmente während der Amplifikation unter Verwendung von Reporteroligonukleotiden beobachtet wird, welche ihre Fluoreszenzeigenschaften durch Interaktion mit dem Amplifikat und/oder der Polymerase ändern.

33. Verfahren nach Anspruch 32, **dadurch gekennzeichnet, dass** man zusätzlich zu dem Reporteroligonukleotid ein weiteres mit einem Fluoreszenzfarbstoff markiertes Oligomer verwendet, welches unmittelbar benachbart zu dem Reporteroligonukleotid an das Amplifikat hybridisiert und sich diese Hybridisierung mittels Fluoreszenz Resonanz Energietransfer nachweisen lässt.

34. Verfahren nach einem der Ansprüche 32 oder 33, **dadurch gekennzeichnet, dass** ein Taqman-Assay durchgeführt wird.

35. Verfahren nach einem der Ansprüche 32 oder 33, **dadurch gekennzeichnet, dass** ein Lightcycler Assay durchgeführt wird

36. Verfahren nach einem der Ansprüche 32 bis 35, **dadurch gekennzeichnet, dass** die Reporteroligonukleotide mindestens eine Fluoreszenzmarkierung tragen.

37. Verfahren nach einem der Ansprüche 32 bis 36, **dadurch gekennzeichnet, dass** die Reportermoleküle die Amplifikation entweder durch eine Zunahme oder eine Abnahme der Fluoreszenz anzeigen.

38. Verfahren nach Anspruch 37, **dadurch gekennzeichnet, dass** man die Zunahme oder Abnahme der Fluoreszenz direkt zur Analyse verwendet und aus dem Fluorenzenzsignal auf einen Methylierungszustand der zu analysierenden DNA schließt.

39. Verwendung eines Verfahrens nach einem der voranstehenden Ansprüche zur Diagnose und/oder Prognose nachteiliger Ereignisse für Patienten oder Individuen, wobei diese nachteiligen Ereignisse mindestens einer der folgenden Kategorien angehören: unerwünschte Arzneimittelwirkungen; Krebserkrankungen; CNS-Fehlfunktionen, Schäden oder Krankheit; Aggressionssymptome oder Verhaltensstörungen; klinische, psychologische und soziale Konsequenzen von Gehimschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion.

40. Verwendung eines Verfahrens nach den Ansprüchen 1-38 zur Unterscheidung von Zelltypen oder Geweben oder zur Untersuchung der Zelldifferenzierung.

## Claims

1. A process for the detection of cytosine methylation in DNA samples, **characterized in that** the following process steps are performed:
a) a genomic DNA sample is treated chemically, preferably with a bisulphite (=disulphite, hydrogen sulphite), in such a way that cytosine is converted into a base different with regard to the base-pairing behaviour in the DNA duplex while 5-methylcytosine remains unchanged,
b) sections of the sample DNA are amplified by means of a polymerase reaction,
c) the DNA is enzymatically selectively cut at such positions that had a methylation status in the DNA sample which is characteristic for the background DNA but not for the DNA to be examined further,
d) the non-enzymatically cut DNA is amplified in another polymerase reaction; through this, the DNA to be examined is enriched in relation to any present background DNA,
e) the amplificate is examined with regard to its sequence and the methylation status in the DNA to be examined in the genomic DNA sample is implied on the basis of this examination.

2. The process according to claim 1, **characterized in that** the sample DNA is obtained from serum or other bodily fluids of an individual,

3. The process according to claim 1, **characterized in that** the sample DNA is obtained from cell lines, blood, sputum, stool, urine, serum, liquor, tissue embedded in paraffin, for example tissue from the eyes, intestine, kidney, brain, heart, prostate, lung, chest or liver, histology slides and any possible combinations thereof.

4. The process according to one of the preceding claims, **characterized in that** the chemical treatment is performed with a bisulphite (=disulphite, hydrogen sulphite).

5. The process according to claim 4, **characterized in that** the chemical treatment is performed after embedding the DNA in agarose.

6. The process according to claim 4, **characterized in that** a reagent denaturing the DNA duplex and/or a radical scavenger is present in the chemical treatment.

7. The process according to one of the preceding claims, **characterized in that** the amplification of several fragments is performed in a reaction vessel in the form of a multiplex PCR.

8. The process according to one of the preceding claims, **characterized in that** the primers used in the amplification amplify the DNA chemically converted with bisulphite but not the corresponding unconverted genomic sequence.

9. The process according to one of the preceding claims, **characterized in that** the enzymatic cut is performed by means of a restriction endonuclease.

10. The process according to claim 9, **characterized in that** the restriction endonuclease is Mae II, Psp 1406 I, Ast II, Ssp 5230 I, Bbr P I, Bsa Al, Sna B I, Cfo I, Hin P1 I, Eco 47 III, NAR I, Ehe I, Kas I, Bbe I, Hae II, Acy I, Ban I, Hgl Cl, Aos I, Avi II, Hpa II, Msp I, Pin Al, Age I, Eco 56 I, Nae I, Cfr10I, SgrAl, Fse I, XmaCl, Sma I, Srf I, Ava I, Bse Al, Mro I, Taq I, Cla I, Sal I, Hind II, Acc I, Xho I, Sfu I, BstBl, Hinf I, Sau 96 I, Dra II, PssI, Ita I, Dsa V, Scr F I, Mae III, Bst E II, Dde I, Cel II, Psp I or Aoc I.

11. The process according to claim 9, **characterized in that** several restriction endonucleases are employed.

12. The process according to claim 11, **characterized in that** several different restriction endonucleases are employed in a reaction vessel.

13. The process according to one of the preceding claims, **characterized in that** the same primers are used in the second amplification step as in the first amplification step.

14. The process according to one of the preceding claims, **characterized in that** primers are additionally or exclusively used in the second amplification step which hybridise to the amplificates from the first step but are not substantially identical or hybridise with the primers of the first step (nested PCR).

15. The process according to one of the preceding claims, **characterized in that** the second amplification step is also performed as a multiplex PCR.

16. The process according to one of the preceding claims, **characterized in that** primers of the second amplification step overlap with the restriction sites of the restriction endonuclease(s) used in the preceding step.

17. The process according to one of the preceding claims, **characterized in that** the background DNA is present in a 100-fold concentration in comparison to the DNA to be examined.

18. The process according to one of the preceding claims, **characterized in that** the background DNA is present in a 1000-fold concentration in comparison to the DNA to be examined.

19. The process according to one of the preceding claims, **characterized in that** the analysis of the sequence properties of the amplificates is performed by means of hybridisation to oligomer arrays wherein the oligomers can be nucleic acids or molecules similar in terms of their hybridisation properties, such as PNAs.

20. The process according to claim 19, **characterized in that** the oligomers hybridise to the DNA to be analysed through a section with a length of 12-22 bases and comprise a CG, TG or CA dinucleotide.

21. The process according to one of the claims 19 or 20, **characterized in that** the methylation status of more than 10 methylation positions of the DNA to be analysed is detected in an experiment.

22. The process according to one of the claims 19 or 20, **characterized in that** the methylation status of more than 60 methylation positions of the DNA to be analysed is detected in an experiment.

23. The process according to one of the claims 1 to 18, **characterized in that** the analysis is performed by sequencing, sequencing methods comprising the Sanger method, Maxam-Gilbert method and other methods, such as Sequencing by Hybridisation (SBH).

24. The process according to claim 23, **characterized in that** the sequencing for each or a small group of CpG positions is performed in each case with a separate primer oligonucleotide and the extension of the primers only accounts for one or a few bases and the methylation status of the respective positions in the DNA to be examined is implied on the basis of the type of the primer extension.

25. The process according to one of the preceding claims, **characterized in that** the existence of a disease or another medical condition of the patient is implied on the basis of the degree of methylation at the different examined CpG positions.

26. The process according to one of the preceding claims, **characterized in that** the amplificates themselves are provided with a detectable label for the detection, the marker preferably being introduced by labelling either the primers or else the nucleotides during the amplification.

27. The process according to claim 26, **characterized in that** the labels are fluorescent labels.

28. The process according to claim 26, **characterized in that** the labels are radionuclides.

29. The process according to claim 26, **characterized in that** the labels are detachable mass labels which are detected in a mass spectrometer.

30. The process according to one of the preceding claims, **characterized in that** in at least one of the amplifications, one of the respective primers is bound to a solid phase.

31. The process according to one of the claims 1 to 18, **characterized in that** the amplificates are collectively detected in the mass spectrometer and thus are uniquely **characterized by** their mass.

32. The process according to one of the claims 1 to 18, **characterized in that** the formation of specific fragments are observed during the amplification by using reporter oligonucleotides which change their fluorescence properties through interaction with the amplificate and/or the polymerase.

33. The process according to claim 32, **characterized in that** another oligomer labelled with a fluorescent dye is used additionally to the reporter oligonucleotide, the oligomer hybridising to the amplificate directly adjacent to the reporter oligonucleotide and it being possible to detect this hybridisation by means of fluorescence resonance energy transfer.

34. The process according to one of the claims 32 or 33, **characterized in that** a TaqMan assay is performed.

35. The process according to one of the claims 32 or 33, **characterized in that** a LightCycler assay is performed.

36. The process according to one of the claims 32 to 35, **characterized in that** the reporter oligonucleotides carry at least one fluorescent label.

37. The process according to one of the claims 32 to 36, **characterized in that** the reporter molecules indicate the amplification by either an increase or a decrease in fluorescence.

38. The process according to claim 37, **characterized in that** the increase or decrease in fluorescence is directly used for the analysis and a methylation state of the DNA to be analysed is implied on the basis of the fluorescence signal.

39. Use of a process according to one of the preceding claims for the diagnosis and/or prognosis of adverse events for patients or individuals, these adverse events belonging to at least one of the following categories: undesirable drug effects; cancer diseases; CNS dysfunctions, defects or disease; aggression symptoms or behaviour disorders; clinical, psychological or social consequences of brain damages; psychotic and personality disorders; dementia and/or associated syndromes; cardiovascular disease, dysfunction and damage; dysfunction of, damage to or disease of the gastrointestinal tract; dysfunction of, damage to or disease of the respiratory system; injury, inflammation, infection, immunity and/or convalescence; dysfunction of, damage to or disease of the body as an anomaly in the development process; dysfunction of, damage to or disease of the skin, muscles, connective tissue or bones; endocrine and metabolic dysfunction, damage or disease; headache or sexual dysfunction,

40. The use of a method according to the claims 1-38 for the discrimination of cell types or tissues or for the examination of cell differentiation.

## Revendications

1. Procédé de mise en évidence de méthylation de la cytosine dans des échantillons d'ADN, **caractérisé en ce que** les étapes de procédé suivantes sont exécutées :
a) un échantillon d'ADN génomique est traité chimiquement, de préférence avec un bisulfite (= bisulfite, hydrogénosulfite) de manière à ce que la cytosine soit transformée en une base différente en ce qui concerne le comportement d'appariement de bases dans le duplex d'ADN, tandis que la 5-méthylcytosine reste inchangée,
b) des sections de l'ADN de l'échantillon sont amplifiées au moyen d'une réaction de polymérase,
c) l'ADN est coupé enzymatiquement de manière sélective aux positions qui présentaient dans l'échantillon d'ADN un état de méthylation qui est caractéristique pour l'ADN de fond, mais pas pour l'ADN à analyser,
d) l'ADN coupé non enzymatiquement est amplifie dans une autre réaction de polymérase, ce par quoi l'ADN à analyser est enrichi par rapport à l'ADN de fond éventuellement existant,
e) l'amplificat est analysé quant à sa séquence, et on en déduit l'état de méthylation dans l'ADN à analyser dans l'échantillon d'ADN génomique.

2. Procédé selon la revendication 1, **caractérisé en ce que** l'on obtient les échantillons d'ADN du sérum ou d'autres fluides corporels d'un individu.

3. Procédé selon la revendication 1, **caractérisé en ce que** l'on obtient les échantillons d'ADN de lignées cellulaires, du sang, du sputum, des selles, de l'urine, de liquide cérébro-spinal, de tissu inclus en paraffine, par exemple tissu des yeux, de l'intestin, du rein, du cerveau, du coeur, de la prostate, du poumon, de la poitrine ou du foie, de porte-objets histologiques et de toutes les combinaisons de ces derniers.

4. Procédé selon l'une quelconque des revendications précédentes, **caractérisé en ce que** l'on réalise le traitement chimique avec un bisulfite (= disulfite, hydrogénosulfite).

5. Procédé selon la revendication 4, **caractérisé en ce que** le traitement chimique est réalisé après inclusion de l'ADN dans l'agarose.

6. Procédé selon la revendication 4, **caractérisé en ce que** le traitement chimique est réalisé en présence d'un réactif dénaturant du duplex d'ADN et/ou d'un capteur radicalaire.

7. Procédé selon l'une quelconque des revendications précédentes, **caractérisé en ce que** l'amplification de plusieurs fragments est réalisée dans un récipient de réaction sous forme de PCR multiplex.

8. Procédé selon l'une quelconque des revendications précédentes, **caractérisé en ce que** les amorces utilisées dans l'amplification amplifient l'ADN transformé chimiquement avec du bisulfite, mais pas la séquence génomique correspondante non transformée.

9. Procédé selon l'une quelconque des revendications précédentes, **caractérisé en ce que** la coupe enzymatique est réalisée au moyen d'une endonucléase de restriction.

10. Procédé selon la revendication 9, **caractérisé en ce que** l'endonucléase de restriction est Mae 11, Psp 1406 I, Ast II, Ssp 5230 I, Bbr P I, Bsa Al, Sna B I, Cfo I, Hin P1 I, Eco 47 III, NAR I, Ehe I, Kas I, Bbe I, Hae II, Acy I, Ban I, Hgi Cl, Aos I, Avi II, Hpa II, Msp I, Pin Al, Age I, Eco 56 I, Nae I, CfrlOl, SgrAl, Fse I, XmaCl, Sma I, Srf I, Ava I, Bse Al, Mro I, Taq I, Cla I, Sal I, Hind II, Ace I, Xho I, Sfu I, BstBl, Hinf I, Sau 96 I, Dra II, Pssl, Ita I, Dsa V, Scr F I, Mae III, Bst E II, Dde I, Cel II, Esp I ou Aoc I.

11. Procédé selon la revendication 9, **caractérisé en ce que** plusieurs endonucléases de restriction sont appliquées.

12. Procédé selon la revendication 11, **caractérisé en ce que** plusieurs endonucléases de restriction différentes sont appliquées dans un récipient de réaction.

13. Procédé selon l'une quelconque des revendications précédentes, **caractérisé en ce qu'**on utilise à la deuxième étape d'amplification les mêmes amorces qu'à la première étape d'amplification.

14. Procédé selon l'une quelconque des revendications précédentes, **caractérisé en ce qu'**on utilise à la deuxième étape d'amplification additionnellement ou exclusivement des amorces qui s'hybrident sur les amplificats de la première étape, mais ne sont pas essentiellement identiques aux amorces de la première étape ou ne s'hybrident pas avec ces derniers (PCR nichée).

15. Procédé selon l'une quelconque des revendications précédentes, **caractérisé en ce que** la deuxième étape d'amplification est également réalisée comme PCR multiplex.

16. Procédé selon l'une quelconque des revendications précédentes, **caractérisé en ce que** des amorces de la deuxième étape d'amplification se chevauchent avec les points de jonction de la ou des endonucléase(s) de restriction appliquée(s) à l'étape précédente.

17. Procédé selon l'une quelconque des revendications précédentes, **caractérisé en ce que** l'ADN de fond est disponible à une concentration 100 fois plus grande que celle de l'ADN à analyser.

18. Procédé selon l'une quelconque des revendications précédentes, **caractérisé en ce que** l'ADN de fond est disponible à une concentration 1000 fois plus grande que celle de l'ADN à analyser.

19. Procédé selon l'une quelconque des revendications précédentes, **caractérisé en ce que** l'analyse des propriétés de séquence des amplificats est réalisée au moyen d'hybridation sur des puces d'oligomères, les oligomères pouvant être des acides nucléiques ou des molécules similaires quant à leurs propriétés d'hybridation comme les APN.

20. Procédé selon la revendication 19, **caractérisé en ce que** les oligomères s'hybrident sur une section d'une longueur de 12-22 bases sur l'ADN à analyser et **en ce qu'**ils comprennent un dinucléotide CG, TG ou CA.

21. Procédé selon l'une quelconque des revendications 19 ou 20, **caractérisé en ce que** l'état de méthylation de plus de 10 positions de méthylation de l'ADN à analyser est détecté dans le cadre d'une expérience.

22. Procédé selon l'une quelconque des revendications 19 ou 20, **caractérisé en ce que** l'état de méthylation de plus de 60 positions de méthylation de l'ADN à analyser est détecté dans le cadre d'une expérience.

23. Procédé selon l'une quelconque des revendications 1 à 18, **caractérisé en ce que** l'analyse est réalisée par séquençage, les méthodes de séquençage comprenant la méthode Sanger, la méthode Maxam-Gilbert et d'autres méthodes comme le séquençage par hybridation (Sequencing by Hybridisation - SBH).

24. Procédé selon la revendication 23, **caractérisé en ce que** l'on réalise le séquençage pour chacune des ou un petit groupe de positions CpG avec chaque fois un oligonucléotide d'amorce séparé et la rallonge des amorces représente seulement une ou juste quelques bases, et on déduit du type de rallonge d'amorce l'état de méthylation des positions concernées dans l'ADN à analyser.

25. Procédé selon l'une quelconque des revendications précédentes, **caractérisé en ce que** l'on déduit du degré de méthylation aux différentes positions CpG analysées la présence d'une maladie ou d'un autre état médical du patient.

26. Procédé selon l'une quelconque des revendications précédentes, **caractérisé en ce que** les amplificats eux-mêmes sont dotés pour la détection d'au moins un repère décelable qui est appliqué de préférence par marquage soit des amorces soit des nucléotides pendant l'amplification.

27. Procédé selon la revendication 26, **caractérisé en ce que** les marquages sont des marquages fluorescents.

28. Procédé selon la revendication 26, **caractérisé en ce que** les marquages sont des radionucléides.

29. Procédé selon la revendication 26, **caractérisé en ce que** les marquages sont des marquages de masse détachables qui sont mis en évidence dans un spectromètre de masse.

30. Procédé selon l'une quelconque des revendications précédentes, **caractérisé en ce que** l'une des amorces est chaque fois liée à une phase solide à au moins une des amplifications.

31. Procédé selon l'une quelconque des revendications 1 à 18, **caractérisé en ce que** les amplificats sont mis en évidence en tout dans le spectromètre de masse et, ainsi, sont **caractérisés** sans équivoque par leur masse.

32. Procédé selon l'une quelconque des revendications 1 à 18, **caractérisé en ce que** la formation de fragments spécifiques pendant l'amplification est observée avec utilisation d'oligonucléotides rapporteurs qui changent leurs propriétés fluorescentes par interaction avec l'amplificat et/ou la polymérase.

33. Procédé selon la revendication 32, **caractérisé en ce que** l'on utilise en plus de l'oligonucléotide rapporteur un autre oligomère marqué avec un colorant fluorescent qui s'hybride sur l'amplificat directement au voisinage de l'oligonucléotide rapporteur et **en ce que** cette hybridation peut être détectée au moyen de transfert d'énergie de résonance de fluorescence.

34. Procédé selon l'une quelconque des revendications 32 ou 33, **caractérisé en ce qu'**un essai Taqman est réalisé.

35. Procédé selon l'une quelconque des revendications 32 ou 33, **caractérisé en ce qu'**un essai Lightcycler est réalisé.

36. Procédé selon l'une quelconque des revendications 32 à 35, **caractérisé en ce que** les oligonucléotides rapporteurs portent au moins un marquage fluorescent.

37. Procédé selon l'une quelconque des revendications 32 à 36, **caractérisé en ce que** la molécule rapporteur indique l'amplification soit par augmentation soit par réduction de la fluorescence.

38. Procédé selon la revendication 37, **caractérisé en ce que** l'on utilise l'augmentation ou la réduction de la fluorescence directement pour l'analyse et déduit du signal fluorescent un état de méthylation de l'ADN à analyser.

39. Utilisation d'un procédé selon l'une quelconque des revendications précédentes pour le diagnostic et/ou le pronostic d'événements désavantageux pour des patients ou des individus, ces événements désavantageux appartenant au moins à l'une des catégories suivantes : effets de médicaments non souhaités ; cancers ; dysfonctionnements, troubles ou maladie du SNC ; symptômes d'agression ou troubles du comportement ; conséquences cliniques, psychologiques et sociales de dommages au cerveau ; troubles psychotiques et troubles de la personnalité ; démence et/ou syndromes associés ; maladies, dysfonctionnement et dommage cardiovasculaire; dysfonctionnement, trouble ou maladie du tractus gastro-intestinal; dysfonctionnement, dommage ou maladie du système respiratoire ; blessure, inflammation, infection, immunité et/ou convalescente ; dysfonctionnement, dommage ou maladie du corps comme déviation dans le processus de développement ; dysfonctionnement, dommage ou maladie de la peau, des muscles, du tissu conjonctif ou des os ; dysfonctionnement, dommage ou maladie endocrinien(ne) et métabolique céphalées ou dysfonctionnement sexuel.

40. Utilisation d'un procédé selon l'une quelconque des revendications 1-38 pour distinguer les types de cellules ou les tissus ou pour analyser la différenciation cellulaire.


### SEQUENZPROTOKOLL

<110> Epigenomics AG
<120> verfahren zum Nachweis von cytosin-Methylierungen mit hoher sensitivität
<130> E01/1310/WO
<140>
   <141>
<160> 4
<170> PatentIn ver. 2.1
<210> 1
   <211> 28
   <212> DNA
   <213> Künstliche Sequenz
<220>
   <223> Beschreibung der künstlichen Sequenz:Primer
<400> 1
   atggttttgt ttaatygtag agttgttt 28
<210> 2
   <211> 27
   <212> DNA
   <213> Künstliche Sequenz
<220>
   <223> Beschreibung der künstlichen sequenz:Primer
<400> 2
   taaacccraa aaaaaaaaac ccaatat 27
<210> 3
   <211> 24
   <212> DNA
   <213> Künstliche Sequenz
<220>
   <223> Beschreibung der künstlichen Sequenz:Primer
<400> 3
   tttattttta tataagtttt gttt 24
<210> 4
   <211> 21
   <212> DNA
   <213> Künstliche Sequenz
<220>
   <223> Beschreibung der künstlichen sequenz:Primer
<400> 4
   cccttcccta caaaactata c 21